(19) Europäisches Patentamt
European Patent Office
Office européen des brevets

(11) Publication number: **0 531 219 B1**

(12) # EUROPEAN PATENT SPECIFICATION

(45) Date of publication of patent specification: **06.12.95**

(51) Int. Cl.6: **A61K 31/415**, C07D 233/64

(21) Application number: **92402418.5**

(22) Date of filing: **04.09.92**

(54) **Use of histamine derivatives for the preparation of drugs, new histamine derivatives and drugs.**

(30) Priority: **06.09.91 US 754914**

(43) Date of publication of application:
**10.03.93 Bulletin 93/10**

(45) Publication of the grant of the patent:
**06.12.95 Bulletin 95/49**

(84) Designated Contracting States:
**AT BE CH DE DK ES FR GB GR IE IT LI LU NL PT SE**

(56) References cited:
**EP-A- 0 420 396**
**EP-A- 0 458 661**
**GB-A- 1 296 544**
**US-A- 3 759 944**
**US-A- 3 891 764**
**US-A- 4 000 302**

(73) Proprietor: **SOCIETE CIVILE BIOPROJET**
**30, rue des Francs Bourgeois**
**F-75003 Paris (FR)**

Proprietor: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventor: **Schwartz, Jean Charles**
**9 Villa Seurat**
**F-75014 Paris (FR)**
Inventor: **Garbarg, Monique**
**26 Boulevard Gouvion Saint Cyr**
**F-75017 Paris (FR)**
Inventor: **Arrang, Jean Michel**
**11 Résidence du Chateau de Courcelles**
**F-91190 Gif sur Yvette (FR)**
Inventor: **Ganellin, Charon Robin**
**Kinwood Briary Wood End,**
**Welwyn**
**Hertfordshire AL6 0TD (GB)**
Inventor: **Lecomte, Jeanne Marie**
**30 rue des Francs-Bourgeois**
**F-75003 Paris (FR)**
Inventor: **Fkyerat, Abdellatif**
**85 rue des Fossés**
**F-59480 La Bassee (FR)**

(74) Representative: **Bernasconi, Jean et al**
**c/o Cabinet Lavoix,**
**2, Place d'Estienne d'Orves**
**F-75441 Paris Cédex (FR)**

EP 0 531 219 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the therapeutic use of histamine derivatives, new histamine derivatives and the use of these derivatives for the preparation of drugs.

In 1983 Arrang et al. (Nature, 1983, 302, 832) detected the existence of a third histamine receptor called $H_3$.

European patent application EP-A-0 420 396, teaches that S-[2-(4(5)-imidazolyl) ethyl] isothiourea and 4-(4(5)-imidazolyl) butyramidine are highly potent selective histamine $H_3$-agonists and provides use of these compounds for treating allergic disease and gastrointestinal motility disorders.

Although it was believed, as a theoretical knowledge, that agonists of the $H_3$-receptor of histamine may inhibit the synthesis and release of neurotransmitters such as histamine in the central nervous system, no individual $H_3$-agonist was ever used for such central indication.

Further it was not apparent that these known compounds may cross the blood-brain barrier and be active in the brain. Also compounds having chemical structures close to these compounds were known to be unable to cross the blood-brain barrier.

Only in EP-A-0 458 661, published after the convention priority date of the present application, it was for the first time disclosed that S-[2-(4(5)-imidazolyl) ethyl] isothiourea and its N-methyl derivative could adequately cross the blood-brain barrier and, further, was a suitable component for preparing an efficient drug to be used as a tranquillizer, sleep-inducer, hypnotic, sedative, and anxiolytic agent.

Treatment of migraine by the usual drugs is often of poor efficiency and is still difficult.

Object of the invention is the use of at last one of the following compounds for the preparation of a drug to be used as an anti-migraine, tranquillizer, sleep-inducer, hypnotic, sedative, anxiolytic, anti-asthmatic and anti-inflamatory agent, notably for the bronchi, the skin or the eyes, or as an anti-gastric ulcer agent:

- Compound A : 4-(4(5)-imidazolyl butyramidine

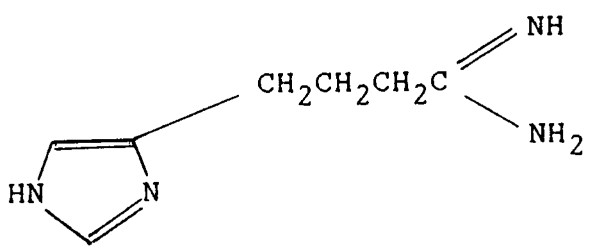

- Compound B : the N-(methyl)butyramidine derivative of compound A
- Compound C : 0-[2-(4(5)-imidazolyl)ethyl] isourea

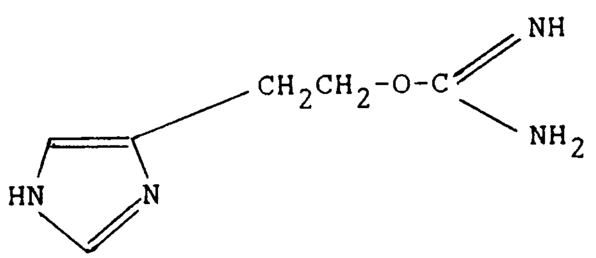

- Compound D : the N-(methyl)isourea derivative of compound C
- Compound E : the S-[2-(4(5)-imidazolyl) ethyl] isothiourea
- Compound F : the N-(methyl)isothiourea derivative of compound E, provided that when compound A is used alone or in combination with compounds E and/or F it is not used as anti-asthmatic, anti-inflammatory or anti-gastric ulcer agent, and
  provided that when compounds E or F are used alone or in combination they are used for the preparation of a drug to be used as an agent against migraine.

Although the prepared agent has gastric (including duodenal) anti-ulcer properties also associated to the action as a peripheral $H_3$ agonist, its efficiency is largely related to the sedative effects on the central nervous systeme.

Another object of the invention is to provide new chemical compounds which are useful to prepare drugs able to cross the blood-brain barrier and to act as histamine agonists on the $H_3$ receptor.

The new compounds according to the invention are selected from the group consisting of compounds B, C and D.

The compound B, which is N-methyl-4[4(5)-imidazolyl] butyramidine, may be prepared from the nitrile of formula

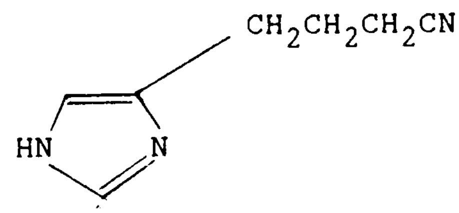

Treatment of the nitrile under anhydrous conditions in the presence of a strong acid (which may be introduced in gaseous form into the reaction mixture) with an alcohol ROH e.g. methanol or ethanol gives an imino-ether of following formula,

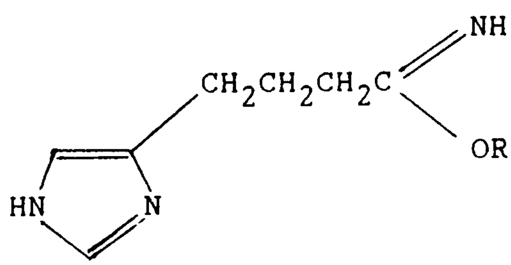

wherein R is, for example, methyl or ethyl, which may be treated with methylamine to yield the required amidine.

Alternatively, treatment of the nitrile at elevated temperature with a methylammonium salt gives the required amidine compound directly.

Example 1 is a particularly preferred embodiment of the method.

Example 1

N-Methyl-4(4(5)-imidazolyl)butyramidine ditrifluoroacetate hydrate.

To a solution of 3-(4(5)-imidazolyl)propionitrile (0.5 g) in absolute ethanol (15 ml) was added dry hydrogen chloride at 0-5 °C for 2 hours and the mixture was allowed to stand for 18 hours in a refrigerator. The solvent was evaporated under reduced pressure and the residual solid was crystallised from ethanol-diethyl ether to afford ethyl 3-(4(5)-imidazolyl)propionimidate dihydrocloride (0.94 g).

A solution of ethyl 3-(4(5)-imidazolyl) propionimidate dihydrochloride (130 mg, 0.5 mmol) in freshly distilled ethanol (5 ml) was chilled to -5 °C in an ice salt bath and treated with 1 ml of ethanolic methylamine solution (methylamine was bubbled for 0.5 minute into ethanol at 0 °C). The solution was stirred at 0 °C for 2 hours and refrigerated overnight. The solvent was evaporated under reduced pressure to afford a solid residue which was chromatographed under high pressure using water containing 1 % methanol and 0.1 % trifluoroacetic acid on a Kromasil $C_{18}$ column to yield the required product as an oil which was dried in vacuo for 16 hours at 40 °C.

$^1$H NMR: ($D_2O$, 400 MHz); δ (ppm) 8.50 (s, 1H, Im-2H); 7.20 (s, 1H, Im-4(5)H); 2.90 (s, 3H, $CH_3$); 2.80 (t, 2H, $CH_2$Amidine); 2.54 (t, 2H, Im-$CH_2$); 2.05 (quint, 2H, $CH_2$).

Mass spectrum : (FAB); m/e 167$(M+H)^+$

| Analysis: | | | |
|---|---|---|---|
| Calc: for $C_8H_{14}N_4$, $2CF_3CO_2H$, $H_2O$,<br>Found: | C 34.96<br>C 35.04 | H 4.40<br>H 4.49 | N 13.59<br>N 13.61 |

Compound C or D of formula

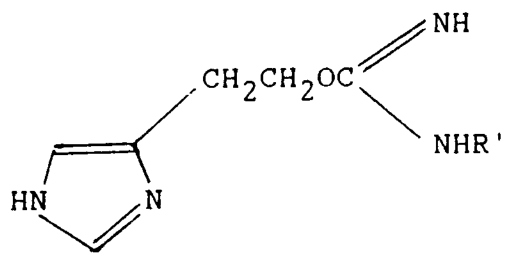

wherein R′ = H or methyl may be prepared by treating cyanamide ($H_2NCN$) or methyl cyanamide ($CH_3$-HNCN) with a hydrohalide salt of the compound 2-[4(5)-imidazolyl] ethan-1-ol (formula hereunder) under anhydrous acidic conditions.

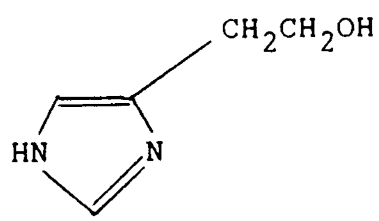

Examples 2 and 3 are particulary preferred embodiments of the method.

Example 2

O-[2-(4(5)-(Imidazolyl)ethyl]isourea dihydrochloride

4(5)-(2-hydroxyethyl)imidazole (0.5 g, 4.46 mmol) and cyanamide (0.375 g, 8.92 mmol) were stirred in benzene (which had been dried over a molecular sieve) (50 ml) saturated with dry hydrogen chloride (until the fumes turned universal indicator paper red) at 20 °C. After 4 days, the mixture reaction was heated at 50 °C. Two days later, chloroform (20 ml) was added, stirring and heating at 50 °C continued for 1 day. Then more hydrogen chloride was added and the mixture heated at 50 °C for a further 7 days. The reaction mixture was then cooled and the solvent decanted. The residue was recrystallised from ethanol: diethyl ether mixture and then recrystallised from ethanol to give the compound named above, m.p. 164-166 °C.

$^1H$ NMR: (DMSO-$d_6$, 400 MHz); δ (ppm) 9.06 (s, 1H, Im-2H); 8.72 (s, 4H, Im-NH and isourea-NH,$NH_2$); 7.53 (s, 1H, Im-4(5)H); 4.53 (t, 2H, $CH_2$-O); 3.10 (t, 3H, Im-CH).

Mass spectrum: (FAB); m/e 155 $(M+H)^+$.

| | | | | |
|---|---|---|---|---|
| Analysis: | Calc: for $C_6H_{10}N_4O$, 2HCl, $0.1C_2H_5OH$ | | | |
| | C 32.14; | H 5.48; | N 24.18; | Cl 30.60 |
| Found : | C 32.24; | H 5.24; | N 24.33; | Cl 30.63 |

Example 3

N-Methyl-O-[2-(4(5)-(Imidazolyl)ethyl]isourea ditrifluoroacetate monohydrate

A solution of cyanogen bromide (5.30 g, 50 mmol) in diethyl ether (dried over sodium) (40 ml) cooled at -3-10 ° C was placed in a three-necked flask fitted with magnetic stirrer and thermometer; dry methylamine gas was bubbled in slowly with stirring and under nitrogen. The bubbling was continued for two hours, keeping the reaction temperature at -3-10 ° C by cooling. At the end of the reaction, the flow of methylamine gas was cut off entirely when the pH of the mixture reached about 7. The resulting mixture was filtered and the ether removed under reduced pressure at low temperature (below 5 ° C because the compound is volatile) in the dark to leave a liquid which was dissolved in ether (5 ml), filtered and evaporated. The residue was recrystallized from ether in a vessel containing a solid carbon dioxide cold finger to give N-methyl cyanamide.

To a suspension of 4(5)-(2-hydroxyethyl)imidazole (0.4 g, 3.6 mmol) in benzene (dried over sodium) (50 ml) saturated with dry hydrogen chloride, was added a solution of an excess of N-methyl cyanamide in benzene (dried over sodium) (20 ml) saturated with dry hydrogen chloride (until the fumes turned universal indicator paper red) at 20 ° C. The reaction mixture was stirred at room temperature for 15 days in a sealed flask. The solvent was decanted and the oily residue was subjected to chromotography under high pressure on a Kromasil $C_{18}$ column using water containing 0.1 % trifluoroacetic acid for elution to yield the product which was isolated as a syrup and was dried in vacuo at 30 ° for 70 hours.

$^1H$ NMR: ($D_2O$, 400 MHz, 75 ° C); δ (ppm) 8.55 (s, 1H, Im-2H); 7.33 (s, 1H, Im-4(5)H); 4.56 (s, 2H, $OCH_3$); 3.25 (s, 2H, $ImCH_2$); 2.85 (s, 3H, $NCH_3$).

Mass spectrum: (FAB); m/e 169 $(M+H)^+$.

| | | | | |
|---|---|---|---|---|
| Analysis: | Calc: for $C_7H_{12}N_4O$, 2.5$CF_3CO_2H$, $H_2O$: | | | |
| | | C 30.58; | H 3.53; | N 11.89; |
| | Found : | C 30.85; | H 3.26; | N 12.29 |

Another object of the invention is a drug comprising an efficient amount of compounds B, C or D which is an hypnotic, sleep-inducer, tranquillizer, sedative or anxiolytic drug or an anti-asthmatic or an anti-inflammatory notably for the bronchi, the skin or the eyes, or an anti-gastric ulcer drug.

These drugs are notably active at a dose between about 0.1 and 30 mg/kg.

A preferred dosage unit for an anti-migraine hypnotic, sleep-inducer, tranquillizer, sedative or anxiolytic drug comprises, in an usual suitable form, from 5 or 6 mg to 500 or 600 mg of compounds A, B, C, D, E or F, where a dosage from 5 or 6 mg to 50 or 60 mg is more preferred for compounds A or C and a dosage of 0.3 to 3 mg/kg, preferably 1 mg/kg is preferred for compounds E and F for oral route administration, and 0.15 to 1.25 mg/kg, preferably 0.5 mg/kg for parenteral route administration, and 5 to 50 mg for intranasal route administration.

The drugs prepared according to the invention may be administered to man in association with a pharmaceutically acceptable excipient or vehicule, as an anti-migraine tranquillizer, sleep-inducer, hypnotic, sedative, anxiolytic anti-asthmatic and anti-inflamatory agent, notably for the bronchi, the skin or the eyes, or as an anti-gastric ulcer agent.

For the preparation of the inventive drugs the compound, once dosed, is mixed with excipients and vehicles as commonly used for the intended oral, parenteral or topical administration.

Of course the above definitions of the compounds encompass obvious equivalents such as their pharmaceutically acceptable salts.

## Claims

1. The use of at least one of the following compounds for the preparation of a drug to be used as an anti-migraine, tranquillizer, sleep-inducer, hypnotic, sedative, anxiolytic, anti-asthmatic and anti-inflamatory agent notably for the bronchi, the skin or the eyes, or as an anti-gastric ulcer agent:

- Compound A : 4-(4(5)-imidazolyl)butyramidine

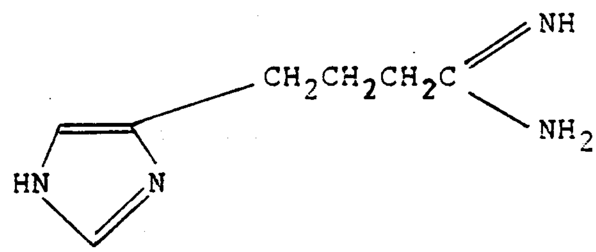

- Compound B : the N-(methyl)butyramidine derivative of compound A
- Compound C : 0-[2-(4(5)-imidazolyl)ethyl] isourea

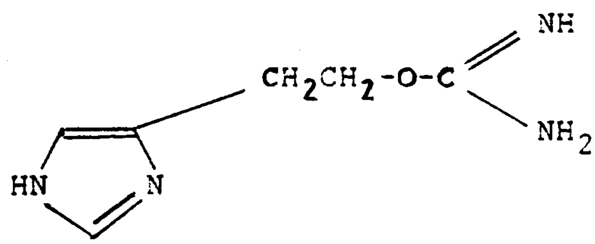

- Compound D : the N-(methyl)isourea derivative of compound C
- Compound E : the S-[2-(4(5)-imidazolyl) ethyl] isothiourea
- Compound F : the N-(methyl)isothiourea derivative of compound E, provided that when compound A is used alone or in combination with compounds E and/or F it is not used as anti-asthmatic, anti-inflammatory or anti-gastric ulcer agent, and
  provided that when compounds E or F are used alone or in combination they are used for the preparation of a drug to be used as an agent against migraine.

**2.** The new compounds selected from the group consisting of compounds B, C and D as defined in claim 1.

**3.** A drug which is active as an hypnotic, sleep-inducer, tranquillizer, sedative, anxiolytic, anti-asthmatic, anti-inflammatory, notably for the bronchi, the skin or the eyes, or as an anti-gastric ulcer agent comprising an efficient amount of the N-(methyl)butyramidine derivative of 4-(4(5)-imidazolyl) butyramidine (B) or the O-[2-(4(5)-imidazolyl)-ethyl] isourea (C) or its N-(methyl)isourea derivative (D).

**4.** A drug according to claim 3 comprising said compound at a dose between 0.1 and 30 mg/kg.

**5.** A drug according to claim 4 comprising said compound at a dose between 5 or 6 mg and 500 or 600 mg.

**Patentansprüche**

**1.** Verwendung mindestens einer der folgenden Verbindungen zur Herstellung eines Arzneimittels, das als Migränemittel, Beruhigungsmittel, Schlafmittel, Hypnotikum, Sedativum, angstlösendes Mittel, Antiasthmatikum und entzündungshemmendes Mittel, insbesondere für Bronchien, Haut oder Augen, oder als Mittel gegen Magengeschwüre Verwendung findet:

- Verbindung A: 4-(4(5)-Imidazolyl)butyramidin

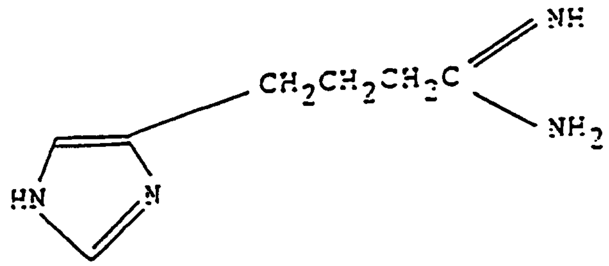

- Verbindung B: das N-(Methyl)butyramidinderivat von Verbindung A
- Verbindung C: O-[2-(4(5)-Imidazolyl)ethyl]isoharnstoff

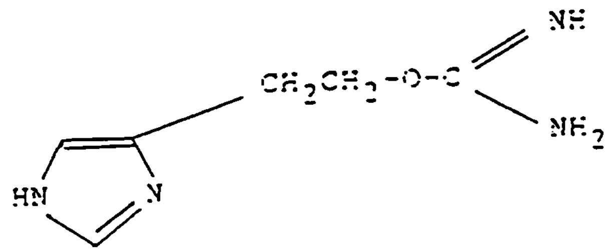

- Verbindung D: das N-(Methyl)isoharnstoffderivat von Verbindung C
- Verbindung E: der S-[2-(4(5)-Imidazolyl)ethyl]isothioharnstoff
- Verbindung F: das N-(Methyl)isothioharnstoffderivat von Verbindung E,
  unter der Voraussetzung, daß bei Verwendung von Verbindung A allein oder in Kombination mit den Verbindungen E und/oder F diese Verbindung A nicht als Antiasthmatikum, entzündungshemmendes Mittel oder Mittel gegen Magengeschwüre verwendet wird, und
  unter der Voraussetzung, daß bei Verwendung von Verbindung E oder F allein oder in Kombination diese für die Herstellung eines Arzneimittels zur Verwendung als Migränemittel verwendet werden.

**2.** Die neuen Verbindungen, ausgewählt aus der Gruppe bestehend aus den wie in Anspruch 1 definierten Verbindungen B, C und D.

**3.** Arzneimittel mit einer ausreichenden Menge an N-(Methyl)butyramidinderivats von 4-(4(5)-Imidazolyl)-butyramidin (B) oder einer ausreichenden Menge an O-[2-(4(5)-Imidazolyl)ethyl]isoharnstoff (C) oder dessen N-(Methyl)isoharnstoffderivat (D), das als Hypnotikum, Schlafmittel, Beruhigungsmittel, Sedativum, angstlösendes Mittel, Antiasthmatikum, entzündungshemmendes Mittel, insbesondere für Bronchien, Haut oder Augen, oder als Mittel gegen Magengeschwüre wirkt.

**4.** Arzneimittel nach Anspruch 3, das diese Verbindung in einer Dosis zwischen 0,1 und 30 mg/kg enthält.

**5.** Arzneimittel nach Anspruch 4, das diese Verbindung in einer Dosis zwischen 5 oder 6 mg und 500 oder 600 mg enthält.

## Revendications

**1.** Utilisation d'au moins un des composés suivants pour la préparation d'un médicament à utiliser comme un agent antimigraineux, tranquillisant, narcotique, hypnotique, sédatif, anxiolytique, anti-asthmatique et anti-inflammatoire, notamment pour les bronches, la peau ou les yeux, ou comme un agent contre les ulcères gastriques :

- Composé A : 4-(4(5)-imidazolyl)butyramidine

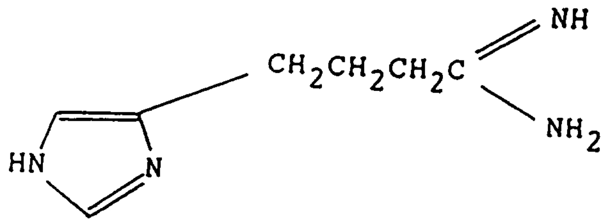

- Composé B : le dérivé N-(méthyl)butyramidine du composé A ;
- Composé C : 0-[2-(4(5)-imidazolyl)éthyl]iso-urée

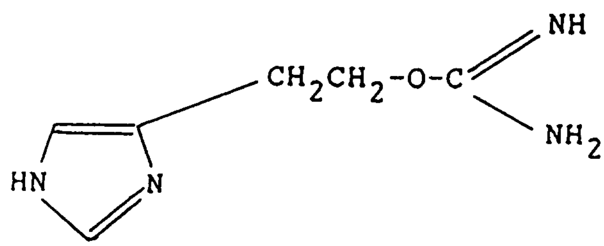

- Composé D : le dérivé N-(méthyl)iso-urée du composé C ;
- Composé E : S-[2-(4(5)-imidazolyl)éthyl]isothio-urée ;
- Composé F : le dérivé N-(méthyl)isothio-urée du composé E,
avec la condition que lorsqu'on utilise le composé A individuellement ou combiné avec les composés E et/ou F, on ne l'utilise pas comme un agent anti-asthmatique, anti-inflammatoire ou comme un agent contre les ulcères gastriques, et avec la condition que lorsqu'on utilise le composé E ou le composé F, individuellement ou combinés, on les utilise pour la préparation d'un médicament destiné à être utilisé comme un agent contre la migraine.

**2.** Nouveaux composés choisis dans l'ensemble constitué par les composés B, C et D tels que définis dans la revendication 1.

**3.** Médicament qui est actif en tant qu'agent hypnotique, narcotique, tranquillisant, sédatif, anxiolytique, anti-asthmatique, anti-inflammatoire, notamment pour les bronches, la peau ou les yeux, ou en tant qu'agent contre les ulcères gastriques, comprenant une quantité efficace du dérivé N-(méthyl)-butyramidine de 4-(4(5)-imidazolyl)butyramidine (B) ou de la O-[2-(4(5)-imidazolyl)éthyl]iso-urée (C) ou de son dérivé N-(méthyl)iso-urée (D).

**4.** Médicament selon la revendication 3, comprenant ledit composé à une dose comprise entre 0,1 et 30 mg/kg.

**5.** Médicament selon la revendication 4, comprenant ledit composé à une dose comprise entre 5 ou 6 mg et 500 ou 600 mg.